(19) Europäisches Patentamt / European Patent Office / Office européen des brevets

(11) **EP 4 205 638 B1**

(12) **EUROPEAN PATENT SPECIFICATION**

(45) Date of publication and mention
of the grant of the patent:
**17.12.2025 Bulletin 2025/51**

(21) Application number: **22175825.3**

(22) Date of filing: **27.05.2022**

(51) International Patent Classification (IPC):
**A61B 5/00** *(2006.01)*    **A61B 5/145** *(2006.01)*
**A61B 5/1455** *(2006.01)*

(52) Cooperative Patent Classification (CPC):
**A61B 5/0075; A61B 5/14532; A61B 5/14546;
A61B 5/1455; A61B 5/7267**

(54) **APPARATUS AND METHOD FOR ESTIMATING TARGET COMPONENT**

VORRICHTUNG UND VERFAHREN ZUR SCHÄTZUNG EINER ZIELKOMPONENTE

APPAREIL ET PROCÉDÉ D'ESTIMATION DE COMPOSANT CIBLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.12.2021 KR 20210189776**

(43) Date of publication of application:
**05.07.2023 Bulletin 2023/27**

(73) Proprietor: **Samsung Electronics Co., Ltd.
Suwon-si, Gyeonggi-do 16677 (KR)**

(72) Inventors:
 • **Choi, Ka Ram**
  Yongin-si (KR)
 • **Lee, June Young**
  Seongnam-si (KR)
 • **Chang, Ho Jun**
  Seoul (KR)

(74) Representative: **Grünecker Patent- und
Rechtsanwälte
PartG mbB
Leopoldstraße 4
80802 München (DE)**

(56) References cited:
 • **GOODARZI M ET AL: "Principal component
analysis-adaptive neuro-fuzzy inference
systems (ANFISs) for the simultaneous
spectrophotometric determination of three
metals in water samples", SPECTROCHIMICA
ACTA PART A: MOLECULAR AND
BIOMOLECULAR SPECTROSCOPY, ELSEVIER,
AMSTERDAM, NL, vol. 73, no. 4, 15 August 2009
(2009-08-15), pages 608 - 614, XP026174134,
ISSN: 1386-1425, [retrieved on 20090326], DOI:
10.1016/J.SAA.2009.03.002**
 • **RUTLEDGE D N ET AL: "Durbin-Watson statistic
as a morphological estimator of information
content", vol. 454, 1 January 2002 (2002-01-01),
pages 277 - 295, XP055981595, Retrieved from
the Internet <URL:https://
sdfestaticassets-eu-west-1.sciencedirectassets.
com/shared-assets/67/images/1px.png?
fr=cpcnjs>**
 • **CENTNER V. ET AL: "Detection of nonlinearity in
multivariate calibration", vol. 376, no. 2, 1
December 1998 (1998-12-01), AMSTERDAM, NL,
pages 153 - 168, XP055981642, ISSN: 0003-2670,
Retrieved from the Internet <URL:https://www.
sciencedirect.com/science/article/pii/
S0003267098005431/pdfft?
md5=a874f23f03e01d15422be45b74402cb7&
pid=1-s2.0-S0003267098005431-main.pdf> DOI:
10.1016/S0003-2670(98)00543-1**

EP 4 205 638 B1

## Description

BACKGROUND

### 1. Field

One or more example embodiments relate to an apparatus and method for non-invasively estimating a target biological component.

### 2. Description of the Related Art

Diabetes is a chronic disease that causes various complications and can be hardly cured, such that people with diabetes are advised to check their blood glucose regularly to prevent complications. In particular, when insulin is administered to control blood glucose, the blood glucose levels have to be closely monitored to avoid hypoglycemia and control insulin dosage. An invasive method of finger pricking is generally used to measure blood glucose levels. However, while the invasive method may provide high reliability in measurement, it may cause pain and inconvenience as well as an increased risk of disease infections due to the use of fingerstick devices. Recently, research has been conducted on a method of non-invasively measuring blood glucose by using a spectrometer without blood sampling.
Goodarzi, M. et al.: "Principal component analysis-adaptive neuro-fuzzy inference systems (ANFISs) for the simultaneous spectrophotometric determination of three metals in water samples", SPECTROCHIMICA ACTA PART A: MOLECULAR AND BIOMOLECULAR SPECTROSCOPY, Elsevier, Amsterdam, NL, vol. 73, no. 4, 15 August 2009 (2009-08-15), pages 608-614, discloses a principal component analysis-adaptive neuro-fuzzy inference system (ANFISs) for the simultaneous spectrophotometric determination of three metals in water samples. Rutledge, D. N. et al.: "Durbin-Watson statistic as a morphological estimator of information content", ANALYTICA CHIMICA ACTA, [Online] vol. 454, 1 January 2002 (2002-01-01), pages 277-295, discloses a method for the evaluation of information content in latent variables, and for the determination of a regression model dimensionality.
It is the object of the present invention to provide an improved method for estimating a target component in a reliable manner.
The invention is claimed in the independent claims. Preferred embodiments are specified in the dependent claims.
According to an aspect of the disclosure, there is provided an apparatus for estimating a target component, the apparatus including: a spectrometer configured to acquire training spectra; and a processor configured to: extract a set number of principal components from the training spectra, determine whether the set number of principal components is appropriate based on randomness of residual in the set number of principal components, and based on the set number of principal components being determined to be appropriate, generate a target component estimation model based on the set number of principal components.
The processor may be further configured to extract the set number of principal components from the training spectra by using at least one of Principal Component Analysis (PCA), Independent Component Analysis (ICA), Non-negative Matrix Factorization (NMF), and Singular Value Decomposition (SVD).
The residual may be a difference between the training spectra, and reconstructed spectra that are reconstructed from the training data based on the set number of principal components.
The processor may be further configured to determine the randomness of the residual, based on a determination of whether the residual in the set number of principal components has a predetermined pattern.
In response to the determination that the residual in the set number of principal components has the predetermined pattern, the processor may be further configured to determine that the residual has no randomness and determine that the set number of principal components is inappropriate; and in response to the determination that the residual in the set number of principal components does not have the predetermined pattern, the processor may be further configured to determine that the residual has the randomness and determines that the set number of principal components is appropriate.
The processor may be further configured to acquire residual spectra by subtracting reconstructed spectra, that are reconstructed based on the set number of principal components, from the training spectra, and determine whether the residual has the predetermined pattern based on a correlation between wavelengths of the residual spectra.
The processor may be further configured to calculate a correlation matrix between the wavelengths of the residual spectra and assign a randomness index to the correlation matrix.
In response to each correlation value in the correlation matrix being greater than or equal to a predetermined value, the processor is further configured to assign an index of 1, and in response to each correlation value in the correlation matrix being less than the predetermined value, the processor is further configured to assign an index of 0.
The processor may be further configured to calculate a total index for the set number of principal components based on the randomness index, and in response to the total index being less than or equal to a predetermined value, the processor may be further configured to determine that the set number of principal components is appropriate.

Based on a determination that the set number of principal components is inappropriate, the processor may be further configured to change the set number of principal components.

By using a determined optimal number of principal components, the processor may be further configured to generate the target component estimation model based on Net Analyte Signal (NAS) calculation.

The spectrometer may be further configured to acquire the training spectra during a training interval, including a fasting period of a user.

The spectrometer may be further configured to acquire an estimation spectrum, and the processor is further configured to estimate the target component by using the target component estimation model and the estimation spectrum, and wherein the target component may include at least one of glucose, urea, lactate, triglyceride, total protein, cholesterol, and ethanol.

According to another aspect of the disclosure, an apparatus for estimating a target biological component, may include: a spectrometer configured to acquire spectra from an object; a memory storing one or more instructions; and a processor configured to execute the one or more instructions to: input the spectra to a target component estimation model, wherein the target component estimation model is trained using a number of principal components that are extracted from training spectra and satisfies a preset randomness criterion of residual; and obtain, as output of the target biological component, an estimated value of the target biological component.

According to another aspect of the disclosure, there is provided a method of estimating a target component, the method including: acquiring training spectra; extracting a set number of principal components from the training spectra; determining whether the set number of principal components is appropriate based on randomness of residual in the set number of principal components; and based on the set number of principal components being determined to be appropriate, generating a target component estimation model based on the set number of principal components.

The determining whether the set number of principal components is appropriate may include determining the randomness of the residual based on whether the residual in the set number of principal components has a predetermined pattern.

The determining of the randomness of the residual based on whether the residual has the predetermined pattern may include: acquiring residual spectra by subtracting reconstructed spectra, that are reconstructed based on the set number of principal components, from the training spectra; and determining whether the residual has the predetermined pattern based on a correlation between wavelengths of the residual spectra.

The determining whether the residual has the predetermined pattern based on the correlation between the wavelengths of the residual spectra may include calculating a correlation matrix between the wavelengths of the residual spectra and assigning a randomness index to the correlation matrix.

The determining whether the set number of principal components is appropriate may include calculating a total index for the set number of principal components based on the randomness index, and in response to the total index being less than or equal to a predetermined value, determining that the set number of principal components is appropriate.

The method may further include, in response to determination that the set number of principal components is inappropriate, changing the set number of principal components.

BRIEF DESCRIPTION OF THE DRAWINGS

The above and/or other aspects will be more apparent by describing certain example embodiments, with reference to the accompanying drawings, in which:

FIG. 1 is a block diagram illustrating an apparatus for estimating a target component according to an example embodiment of the present disclosure;
FIG. 2 is a diagram illustrating a training interval and an estimation interval;
FIGS. 3A and 3B are diagrams illustrating an example of residual spectra obtained by varying a number of principal components;
FIGS. 4A and 4B are diagrams illustrating an example of a correlation matrix;
FIGS. 5A and 5B are diagrams illustrating an example of assigning a randomness index to a correlation matrix;
FIG. 6 is a block diagram illustrating an apparatus for estimating a target component according to another example embodiment of the present disclosure;
FIG. 7 is a flowchart illustrating a method of estimating a target component according to an example embodiment of the present disclosure;
FIG. 8 is a diagram illustrating a wearable device according to an example embodiment of the present disclosure; and
FIG. 9 is a diagram illustrating a smart device according to an example embodiment of the present disclosure.

DETAILED DESCRIPTION

Example embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the example embodiments. However, it is apparent that the example embodiments can be practiced without those specifically defined matters. Also, well-known functions or constructions are not described in detail since they would obscure the description with unnecessary detail.

It will be understood that, although the terms first, second, etc. may be used herein to describe various elements, these elements should not be limited by these terms. These terms are only used to distinguish one element from another. Any references to singular may include plural unless expressly stated otherwise. In addition, unless explicitly described to the contrary, an expression such as "comprising" or "including" will be understood to imply the inclusion of stated elements but not the exclusion of any other elements. Also, the terms, such as "unit" or "module", etc., should be understood as a unit for performing at least one function or operation and that may be embodied as hardware, software, or a combination thereof.

Expressions such as "at least one of," when preceding a list of elements, modify the entire list of elements and do not modify the individual elements of the list. For example, the expression, "at least one of a, b, and c," should be understood as including only a, only b, only c, both a and b, both a and c, both b and c, all of a, b, and c, or any variations of the aforementioned examples.

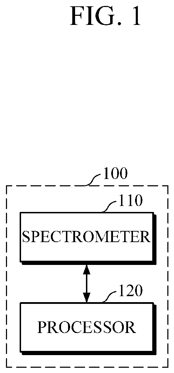

FIG. 1 is a block diagram illustrating an apparatus for estimating a target component according to an example embodiment of the present disclosure.

The apparatus for estimating a target component according to embodiments of the present disclosure is an apparatus for estimating a target component by analyzing spectra of an object and may be mounted in various electronic devices. In this case, examples of the electronic device may include a cellular phone, a smartphone, a tablet PC, a laptop computer, a personal digital assistant (PDA), a portable multimedia player (PMP), a navigation, an MP3 player, a digital camera, a wearable device, etc., and examples of the wearable device may include a wristwatch-type wearable device, a wristband-type wearable device, a ring-type wearable device, a waist belt-type wearable device, a necklace-type wearable device, an ankle band-type wearable device, a thigh band-type wearable device, a forearm band-type wearable device, and the like. However, the electronic device and the wearable device are not limited to the above examples.

Referring to FIG. 1, an apparatus 100 for estimating a target biological component includes a spectrometer 110 and a processor 120.

The spectrometer 110 may acquire spectra of an object. In particular, the spectra of the object may include a reflectance spectrum, a single beam spectrum, a near-infrared absorption spectrum, a mid-infrared absorption spectrum, a visible light absorption spectrum, and the like. For example, the near-infrared absorption spectrum may include a near-infrared absorption spectrum ranging from $4000cm^{-1}$ to $5000cm^{-1}$ or a near-infrared absorption spectrum ranging from $5500cm^{-1}$ to $6500cm^{-1}$, but is not limited thereto.

The spectrometer 110 may acquire a plurality of in vivo spectra (hereinafter referred to as "training spectra") measured during an interval (hereinafter referred to as a "training interval") in which a target component concentration of the object is substantially constant. In particular, the training interval may refer to a fasting period.

In addition, the spectrometer 110 may acquire an in vivo spectrum (hereinafter referred to as an "estimation spectrum") measured during an interval (hereinafter referred to as an "estimation interval") in which a target component concentration of the object changes, for example, when a user consumes food.

Examples of the target component may include glucose, urea, lactate, triglyceride, total protein, cholesterol, collagen, elastin, keratin, ethanol, and the like. However, the target component is not limited thereto, and may include various substances formed by molecular bonds of C-H, N-H, O-H, and the like.

For example, the spectrometer 110 may acquire spectra by emitting light towards the object and receiving light reflected or scattered from the object. In particular, the spectrometer 110 may use Infrared spectroscopy, Raman spectroscopy, etc., but is not limited thereto and may measure spectra by using various spectroscopic techniques.

The spectrometer 110 may include a light source for emitting light towards the object, and a photodetector for measuring spectra by receiving light reflected or scattered from the object.

Here, the light source may be configured to emit visible rays, Near Infrared Rays (NIR), Mid Infrared Rays (MIR), and the like towards the object. However, wavelengths of light emitted by the light source may vary according to the measurement purpose or type of an analyte. Further, the light source is not necessarily a single light-emitting body, but may be formed of an array of a plurality of light-emitting bodies. The light source may include a light emitting diode (LED), a laser diode, a phosphor, and the like.

The photodetector may include a spectrometer which acquires spectra by splitting light reflected or scattered from the object. Alternatively, the photodetector may include a photo diode, a photo transistor, a complementary metal-oxide semiconductor (CMOS) image sensor, a charge-coupled device (CCD) image sensor, and the like. The photodetector is not necessarily a single device, and may be formed of an array of a plurality of devices. There may be various numbers and arrangements of the light source and the photodetector, and the number and arrangement thereof may vary according to the type and purpose of use of an analyte, the size and shape of the electronic device in which the apparatus 100 for estimating a target component is mounted, and the like.

In another example, the spectrometer 110 may receive in vivo spectra from an external device for measuring or storing the in vivo spectra. Here, the external device may include various portable devices, such as a smartphone, a tablet PC, a smart ring, smart earphones, a smart necklace, a smart watch, a smart band, etc., or a server of a specialized medical institution. The spectrometer 110 may communicate with the external device by using various communication techniques, such as Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, Ultra-Wideband (UWB) communication, Ant+ communication, WIFI communication, Radio Frequency Identification (RFID) communication, 3G, 4G, and 5G communications, and the like.

The processor 120 may control the spectrometer 110 to generate a target component estimation model and to estimate a target component, and may receive spectra from the spectrometer 110.

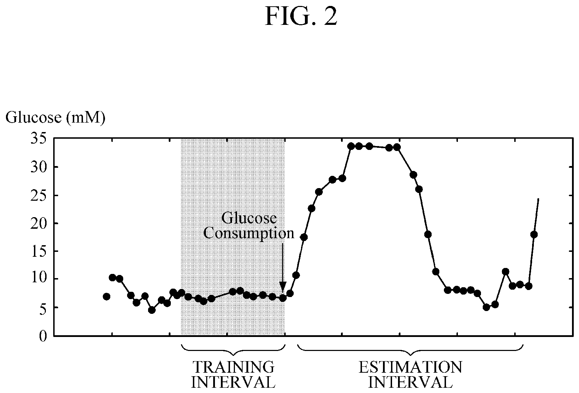

Upon receiving the training spectra, including the plurality of in vivo spectra, from the spectrometer 110, the processor 120 may generate a target component estimation model by using the training spectra. In addition, upon receiving the estimation spectrum including one or more in vivo spectra, the processor 120 may estimate a target component based on the estimation spectrum by using the target component estimation model, which will be described in detail below with reference to FIG. 2. FIG. 2 is a diagram illustrating the training interval and the estimation interval.

Referring to FIG. 2, the processor 120 may generate a target component estimation model by learning a spectrum change factor, which is irrelevant to a change in target component concentration, by using the in vivo spectra measured during the "training interval." In addition, the processor 120 may estimate the target component by using the in vivo spectra, measured in the "estimation interval" following the training interval, and by using the target component estimation model.

That is, the processor 120 may estimate a target component by generating the target component estimation model based on the in vivo spectra measured during the training interval, and then applying the generated target component estimation model to the estimation interval. In particular, when at least one of spectrum change factors, which are irrelevant to a change in target component, e.g., blood glucose concentration, is changed at a point of the estimation interval due to a temperature change of the object, a change in pressure between the object and the apparatus, etc., residual increases starting from that point, thereby increasing an error in estimation of the target component, e.g., blood glucose.

The processor 120 may extract principal components, constituting the object, from the training spectra, and may generate the target component estimation model based on the extracted principal components.

In particular, the processor 120 may extract the principal components by using principal component analysis. However, the processor 120 is not limited thereto and may extract the principal components by using Independent Component Analysis (ICA), Non-negative matrix factorization (NMF), Singular Value Decomposition (SVD), and the like.

The processor 120 may set an arbitrary number of principal components and may determine whether the set number of principal components is appropriate based on randomness of the residual in the set number of principal components.

In particular, the residual may be a difference between spectra, reconstructed based on the set number of principal components, and the training spectra acquired by the spectrometer 110. That is, the residual may be an error measured by comparing the spectrum, reconstructed based on the set number of principal components, with an actually measured in vivo spectrum.

Referring to the following Equation 1, the acquired training spectrum A is composed of a target component signal S to be estimated, a physical noise signal Np, a statistical noise signal Ns on which principal component analysis is performed, and residual E.

[Equation 1]

$$A = S + Np + Ns + E$$

There may be at least two or more acquired training spectra A. The target component signal S to be estimated may refer to, for example, a blood glucose signal and the like. The physical noise signal Np may be a noise signal related to an offset, a slope, an environmental noise (e.g., temperature and humidity), and may be calculated in advance before generating the target component estimation model. The statistical noise signal Ns may be extracted by principal component analysis and the like. The residual E may refer to an error value which is not considered in generating the target component estimation model, as described above.

In particular, the processor 120 may obtain the residual E by subtracting the target component signal S to be estimated, the physical noise signal Np, and the statistical noise signal Ns from the acquired training spectrum A.

The processor 120 may determine randomness of the residual according to the set number of principal components based on whether the residual has a predetermined pattern. In this case, the predetermined pattern may refer to a similarity or difference between residual values of the respective training spectra, an aspect of change in the residual values, and the like.

In particular, if the residual has the predetermined pattern, the processor 120 may determine that the residual has no randomness and may determine that the set number of principal components is inappropriate. By contract, if there is no predetermined pattern in the residual, the processor 120 may determine that the residual has randomness and may determine that the set number of principal components is appropriate.

If modeling is performed by extracting sufficient noise from the training spectra to generate the target component estimation model, an error (residual) between an actual target component value and an estimated target component value shows a random pattern; by contrast, if modeling is performed without extracting sufficient noise, the error (residual) between the actual target component value and the estimated target component value shows a specific pattern. That is, the error (residual) showing a regular pattern provides circumstantial evidence that data are not fitted properly. Accordingly, by determining appropriateness of the set number of principal components based on the randomness of the residual, a target component estimation model may be generated with improved accuracy.

The processor 120 may acquire residual spectra of the respective training spectra and may determine whether there is a pattern in the residual based on the acquired respective residual spectra.

A process of acquiring the respective residual spectra will be described in detail below with reference to the following Equations 2 and 3.

A concentration quantified using linear regression of the training spectra A, acquired by the above Equation 1, may be expressed by the following Equation 2.

[Equation 2]

$$A_i = [\sum_{k=1}^{n_{sign}} \varepsilon_k \cdot L_i \cdot \Delta C_k + \sum_{d=1}^{n_{pysis}} q_d \cdot N_d + \sum_{U=1}^{n_{stat}} p_U \cdot V_U] + E_i$$

$A_i$ denotes an i-th acquired training spectrum; $E_i$ denotes the residual spectrum of the i-th acquired training spectra; $n_{sign}$ denotes the number of target components to be estimated; $n_{psysis}$ denotes the number of physical noises; $n_{stat}$ denotes the set number of principal components; $\varepsilon_k$ denotes a pure unit spectrum for a k-th target component; $L_i$ denotes a light path length of the i-th training spectrum; $\Delta C_k$ denotes a variation in the k-th target component to be estimated; $N_d$ denotes a d-th physical noise signal; $q_d$ denotes a coefficient of a d-th physical noise signal; $V_U$ denotes a U-th principal component; and $p_U$ denotes a coefficient of the U-th principal component.

In particular, the processor 120 may acquire spectra $[\sum_{k=1}^{n_{sign}} \varepsilon_k \cdot L_i \cdot \Delta C_k + \sum_{d=1}^{n_{pysis}} q_d \cdot N_d + \sum_{U=1}^{n_{stat}} p_U \cdot V_U]$ reconstructed according to the set number $n_{stat}$ of principal components by using Equation 2. In the case where the target component is blood glucose, the respective training spectra $A_i$ are acquired during a fasting period, such that the value of $\Delta C_k$ may be ignored; and $\sum_{d=1}^{n_{pysis}} q_d \cdot N_d$ may be calculated in advance before generating the target component estimation model, as described above.

The terms of Equation 2 may be arranged based on the residual spectrum $E_i$ to provide the following Equation 3.

[Equation 3]

$$E_i = A_i - \left[ \sum_{k=1}^{n_{sign}} \varepsilon_k \cdot L_i \cdot \Delta C_k + \sum_{d=1}^{n_{pvsis}} q_d \cdot N_d + \sum_{U=1}^{n_{stat}} p_U \cdot V_U \right]$$

That is, the processor 120 may acquire each residual spectrum $E_i$ by subtracting the spectra $\left[ \sum_{k=1}^{n_{sign}} \varepsilon_k \cdot L_i \cdot \Delta C_k + \sum_{d=1}^{n_{pvsis}} q_d \cdot N_d + \sum_{U=1}^{n_{stat}} p_U \cdot V_U \right]$ , reconstructed according to the set number $n_{stat}$ of principal components, from the acquired respective training spectra $A_i$.

Based on the acquired respective residual spectra $E_i$, the processor 120 may calculate an m x n residual matrix $X_{residual}$, as shown in the following Equation 4.

[Equation 4]

$$X_{residual} = [e_{ij}]_{m \times n}$$

In this case, $e_{ij}$ denotes, for example, a residual value at a wavelength of j of the i-th training spectrum; m denotes the number of spectra; and n denotes the number of wavelengths.

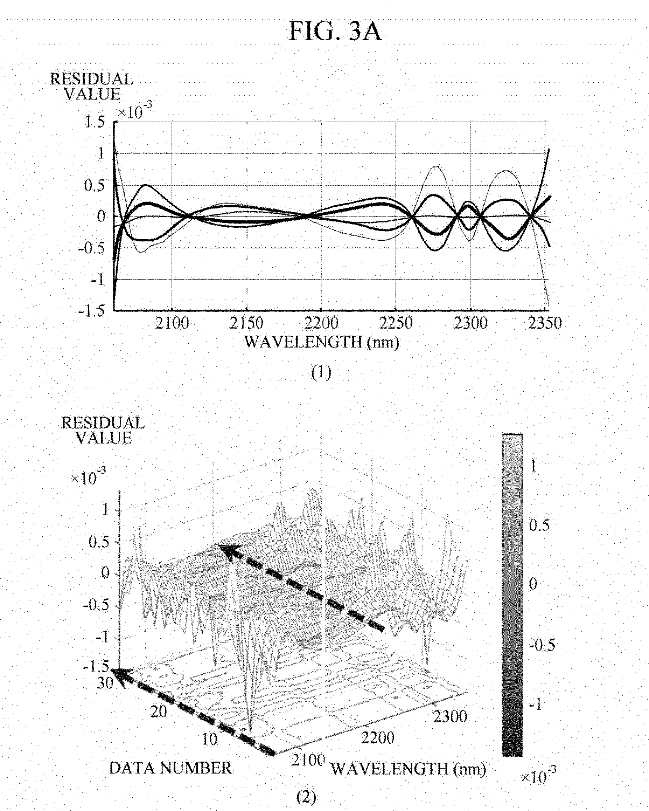

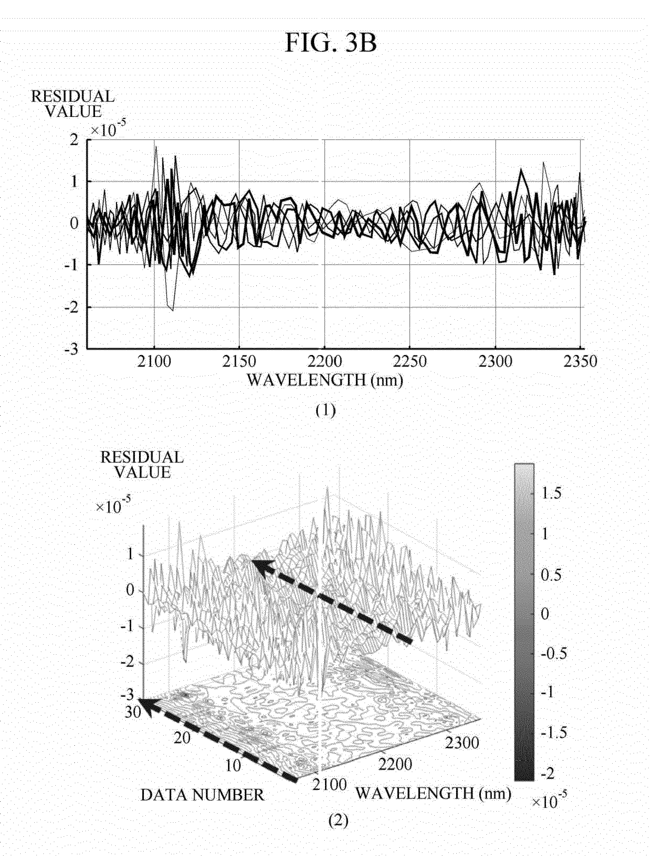

The processor 120 may determine whether the residual has a predetermined pattern based on a correlation between wavelengths of the respective residual spectra, which will be described in detail with reference to FIGS. 3A and 3B.

FIGS. 3A and 3B are diagrams illustrating an example of residual spectra obtained by varying the number of principal components. FIG. 3A (1) and FIG. 3B (1) are 2D views of residual spectra, and FIG. 3A (2) and FIG. 3B (2) are 3D views of residual spectra.

That is, in (2) of FIG. 3A and (2) of FIG. 3B, the respective axes denote a residual value, a data number (training spectrum index over time), and a wavelength (nm), and (1) of FIG. 3A and (1) of FIG. 3B illustrate 2D views of a portion (five spectra) of the plurality of training spectra.

In (1) of FIG. 3A and (1) of FIG. 3B, the horizontal axis denotes a wavelength, and the vertical axis denotes a residual value. While FIGS. 3A and 3B denote five acquired residual spectra, the number is merely an exemplary value and is not limited thereto.

In (1) of FIG. 3A, five residual spectra are acquired by setting the number of principal components to 1 in five training spectra; and in (1) of FIG. 3B, five residual spectra are acquired by setting the number of principal components to 15 in five training spectra.

Referring to (1) of FIG. 3A, it can be seen that when the number of principal components is set to 1, a difference between residual values in the respective residual spectra increases (e.g., a point where the wavelength is 2275 nm) or decreases (e.g., a point where the wavelength is 2185 nm) as the wavelength changes. By contrast, referring to (1) of FIG. 3B, it can be seen that the residual values in the respective residual spectra change very irregularly as the wavelength changes, and there is no pattern in the residual values.

In the case where the number of principal components is set to 1 (FIG. 3A), the processor 120 may determine that the residual has a predetermine pattern; and in the case where the number of principal components is set to 15 (FIG. 3A), the processor 120 may determine that the residual has no predetermine pattern.

Referring back to FIG. 1, the processor 120 may calculate a correlation matrix between wavelengths of the respective residual spectra, and may determine whether the residual has a pattern based on the calculated correlation matrix.

For example, based on the calculated residual matrix as shown in Equation 4, the processor 120 may calculate an n x n correlation matrix, as show in the following Equation 5.

[Equation 5]

$$X_{corr} = [r_{ij}]_{n \times n}$$

$$r_{ij} = \frac{cov(E_i, E_j)}{\sigma E_i \cdot \sigma E_j}$$

Herein, n denotes the number of wavelengths; $E_i$ and $E_j$ denote residual values over time of i-th and j-th wavelengths, respectively; $\sigma E_i$ and $\sigma E_j$ denote standard deviations of $E_i$ and $E_j$; and $cov(E_i, E_j)$ denotes covariances of $E_i$ and $E_j$. The respective correlation values $r_{ij}$ may range from -1 to 1. In this case, $r_{11}$, $r_{22}$, and $r_{33}$, which indicate correlations of the residual values at the same wavelength, have a value of 1.

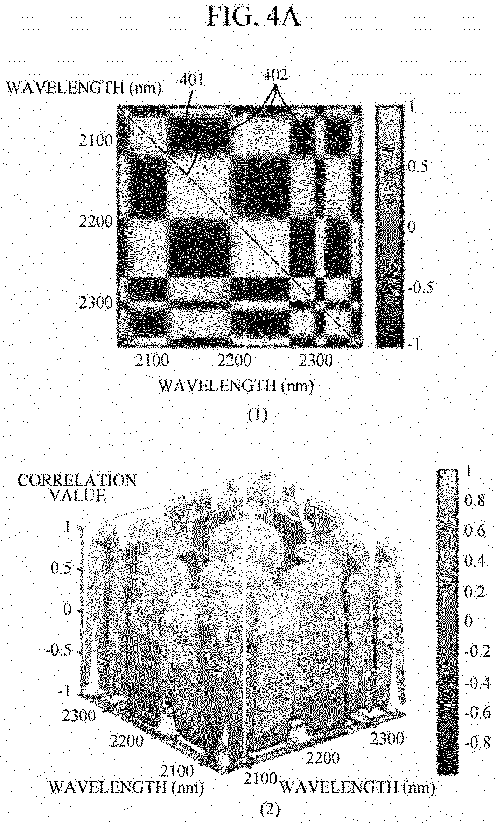

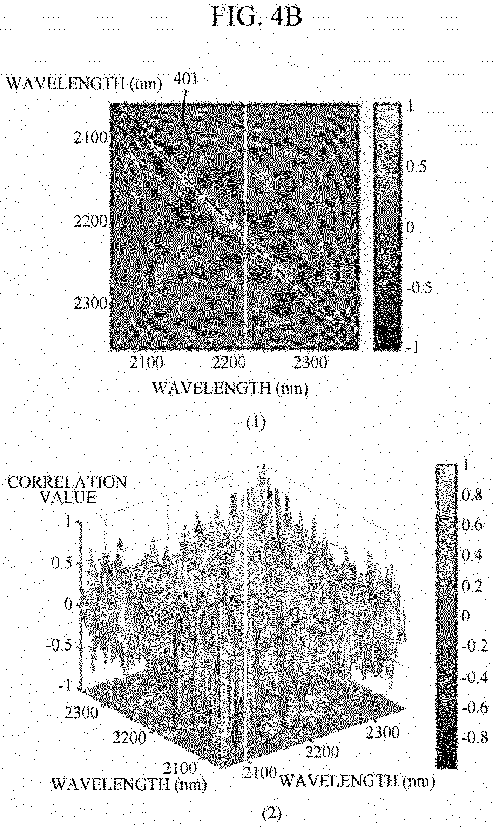

FIGS. 4A and 4B are diagrams illustrating an example of a correlation matrix.

FIG. 4A (1) and FIG. 4B (1) are 2D views of a correlation matrix, and FIG. 4A (2) and FIG. 4B (2) are 3D views of a correlation matrix. In (2) of FIG. 4A and (2) of FIG. 4B, the respective axes denote a wavelength (nm) and a correlation value; and in (1) of FIG. 4A and (1) of FIG. 4B, the correlation values are displayed in shades instead of in 2D.

Referring to (1) of FIG. 4A and (1) of FIG. 4B, a correlation matrix between wavelengths of the respective residual spectra in a predetermined wavelength range of 2100 nm to 2300 nm are shown. In (1) of FIG. 4A and (1) of FIG. 4B, the values of the correlation matrix are shown in different shades according to the respective correlation values ranging from -1 to 1. As the correlation increases, i.e., is closer to 1, the values of the correlation matrix are shown in white, and as the correlation decreases, i.e., is closer to -1, the values are shown in black.

FIG. 4A (1) shows a correlation matrix when the number of principal components is set to 1, and FIG. 4B (1) shows a correlation matrix when the number of principal components is set to 15. As described above, in both the cases of FIG. 4A (1) and FIG. 4B (1), a correlation 401 of the residual values at the same wavelength is shown as 1, i.e., in white.

As in the cases of FIG. 3A (1) and FIG. 3B (1), it can be seen that in the case where the number of principal components is set to 1 (FIG. 4A (1)), there are more data shown in white, i.e., data having correlation values close to 1, when compared to the case of FIG. 4B (1). In addition, in the correlation matrix of FIG. 4A (1), a very high correlation value and a very low correlation value are separated by a specific correlation value (e.g., 402) according to a change in wavelength. This indicates that there is a very high correlation between the respective residual values according to wavelengths, and thus the residual has a predetermined pattern.

In particular, the processor 120 may also determine that the residual has a predetermined pattern when the number of principal components is set to 1 (FIG. 4A), and even when the number of principal components is set to 15 (FIG. 4B), the processor 120 may also determine that the residual has no predetermined pattern.

Referring back to FIG. 1, the processor 120 may assign a randomness index to the calculated correlation matrix.

For example, if the respective correlation values $r_{ij}$ in Equation 5 or the respective correlation values in FIGS. 4A and 4B are greater than or equal to a predetermined value, the processor 120 may assign an index of 1, and if the values are smaller than the predetermined value, the processor 120 may assign an index of 0. In this case, the predetermined value may be 0.7, but is not limited thereto and may be changed to other values.

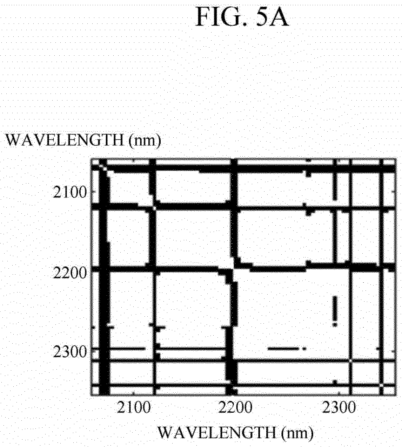

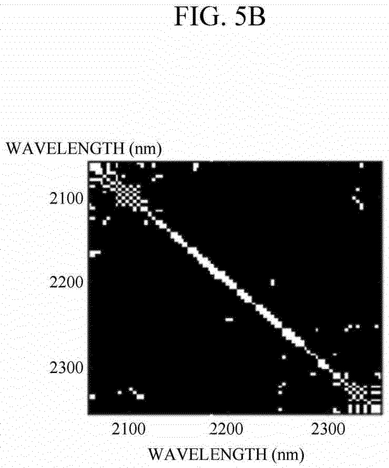

A result of assigning a randomness index to the calculated correlation matrix is illustrated in FIGS. 5A and 5B.

FIGS. 5A and 5B are diagrams illustrating an example of assigning a randomness index to a correlation matrix.

FIG. 5A illustrates a result of assigning an index when the number of principal components is set to 1, and FIG. 5B illustrates a result of assigning an index when the number of principal components is set to 15. Referring to FIGS. 5A and 5B, in the case where the randomness index is 1, the correlation matrix is shown in white, and in the case where the randomness index is 0, the correlation matrix is shown in black.

In the case where the number of principal components is set to 1 (FIG. 5A), there are more values having the randomness index of 1 (shown in white) than the case where the number of principal components is set to 15 (FIG. 5B).

That is, in the case where the number of principal components is set to 1 (FIG. 5A), there are more correlation values greater than or equal to a predetermined value (e.g., 0.7) than the case where the number of principal components is set to 15 (FIG. 5B). Accordingly, when the number of principal components is set to 1 (FIG. 5A), the processor 120 may determine that the residual has a predetermined pattern; and when the number of principal components is set to 15 (FIG. 5B), the processor 120 may determine that the residual has no predetermined pattern.

Referring back to FIG. 1, the processor 120 may assign a randomness index to the calculated correlation matrix and may calculate an index matrix $X_{index}$ of n x n, as shown in the following Equation 6. In this case, $c_{ij}$ denotes an index assigned to each correlation matrix value.

[Equation 6]

$$X_{index} = [c_{ij}]_{n \times n}$$

Based on the randomness index assigned to the correlation matrix, the processor 120 may calculate a total index for the set number of principal components. The following Equation 7 is an example of calculating a total index $idx_{total}$ for the set number of principal components, but the calculation is not limited thereto. In the following Equation 7, $c_{ij}$ denotes a result of assigning an index to each correlation matrix value as described above, and n denotes the number of wavelengths.

[Equation 7]

$$idx_{total} = 100 \times \frac{(\sum_{j=1}^{n} \sum_{i=1}^{n} c_{ij}) - n}{n^2 - n}$$

If the total index calculated for the set number of principal components is smaller than or equal to a predetermined value, the processor 120 may determine that the residual has no pattern and may determine that the set number of principal components is appropriate. By contrast, if the calculated total index exceeds the predetermined value, the processor 120 may determine that there is a pattern in the residual and may determine that the set number of principal components is inappropriate.

Upon determining that the set number of principal components is inappropriate, the processor 120 may reset the number of principal components to extract the principal components.

Unlike the example in FIG. 1 described so far, instead of determining appropriateness of any set number of principal components (e.g., 3), the processor 120 may extract the principal components a plural number of times by varying the number of principal components (e.g., the number of principal components being 1, 10, and 15), and may determine an optimal number of principal components among the numbers.

For example, in the case where the principal components are 1, 10, and 15 in number, the processor 120 may determine an optimal number of principal components by repeating the above process for the respective numbers. For example, by determining whether there is a pattern in the residual obtained for the respective numbers, the processor 120 may determine a number, at which the residual has a minimal pattern, as the optimal number of principal components. In particular, the processor 120 may acquire a plurality of residual spectra for each number of principal components, may calculate a correlation matrix and assign a randomness index thereto, may calculate a total index as shown in Equation 7, and may determine a number (e.g., 15) of principal components, at which the calculated total index is minimized, as the optimal number of principal components. In particular, among the numbers of principal components, at which the calculated total index is smaller than or equal to a predetermined value, the processor 120 may determine a number of principal components, at which the total index is minimized, as the optimal number of principal components.

Upon determining that any set number of principal components is appropriate or determining the optimal number of principal components among the plurality of numbers of principal components, the processor 120 may generate a target component estimation model based on the optimal number of principal components by using linear regression, such as net analyte signal (NAS) calculation, classical least square (CLS), etc., machine learning, the Beer-Lambert law, and the like. The following Equation 8 is an example of the generated target component estimation model but the model is not limited thereto.

[Equation 8]

$$M = \left[ \sum_{k=1}^{n_{sign}} \varepsilon_k, \sum_{d=1}^{n_{pysis}} N_d, \sum_{U=1}^{n_{stat}} V_U \right]$$

$$\Delta C_k = \left[ (M^T \times M)^{-1} \times M^T \right] \times S$$

Herein, M denotes the target component estimation model; $[(M^T \times M)^{-1} \times M^T]$ denotes a pseudo-inverse vector; S denotes a spectrum obtained for estimating a target component; and $\Delta C_k$ denotes a variation in target component compared to a training interval.

Then, once the estimation spectrum (e.g., S in Equation 8) is acquired during the estimation interval as described above, the processor 120 may estimate a target component by applying the acquired estimation spectrum to the target component estimation model (e.g., M in Equation 8).

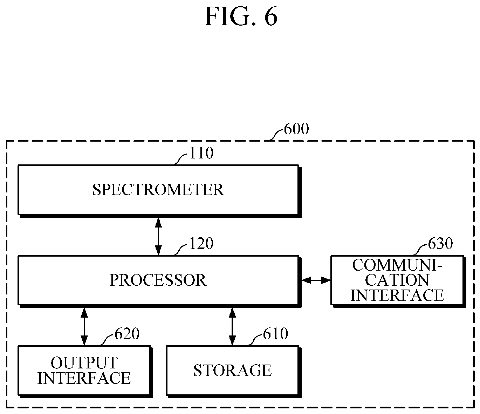

FIG. 6 is a block diagram illustrating an apparatus 600 for estimating a target component according to another example embodiment of the present disclosure.

Referring to FIG. 6, an apparatus 600 for estimating a target component may include the spectrometer 110, the processor 120, a storage 610, an output interface 620, and a communication interface 630, in which the spectrometer 110 and the processor 120 are described in detail above, such that a detailed description thereof will be omitted.

The storage 610 may store programs or commands for operation of the apparatus 600 for estimating a target component, and may store data input to the apparatus 600 for estimating a target component and data related to estimating a target component, e.g., the in vivo spectra, the target component estimation model, the estimated target component values, and the like.

The storage 610 may include at least one storage medium of a flash memory type memory, a hard disk type memory, a multimedia card micro type memory, a card type memory (e.g., an SD memory, an XD memory, etc.), a Random Access Memory (RAM), a Static Random Access Memory (SRAM), a Read Only Memory (ROM), an Electrically Erasable Programmable Read Only Memory (EEPROM), a Programmable Read Only Memory (PROM), a magnetic memory, a magnetic disk, and an optical disk, and the like. Further, the apparatus 600 for estimating a target component may operate an external storage medium, such as web storage and the like, which performs a storage function of the storage 610 on the Internet.

The output interface 620 may output data generated and processed by the spectrometer 110 and/or the processor 120, and the like. For example, the output interface 620 may output the spectra acquired by the spectrometer 110, and/or the estimated target component values obtained by the processor 120, warning information based on whether the target component value is normal, and the like. The output interface 620 may output a target component estimation result and the like by using at least one of an acoustic method, a visual method, and a tactile method. To this end, the output interface 620 may include a display, a speaker, and a haptic module such as a vibrator and the like.

The communication interface 630 may communication with an external device. For example, the communication interface 630 may transmit the data input to the apparatus 600 for estimating a target component, data stored in and processed by the apparatus 600 for estimating a target component, and the like to the external device; or may receive, from the external device, various data related to generating/updating a target component estimation model and estimating a target component.

The external device may be medical equipment using the data input to the apparatus 600 for estimating a target component, the data stored in and processed by the apparatus 600 for estimating a target component, etc., a printer to print out results, or a display to display the results. In addition, the external device may be a digital TV, a desktop computer, a cellular phone, a smartphone, a tablet PC, a laptop computer, a personal digital assistant (PDA), a portable multimedia player (PMP), a navigation, an MP3 player, a digital camera, a wearable device, etc., but is not limited thereto.

The communication interface 630 may communicate with an external device by using Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, Ultra-Wideband (UWB) communication, Ant+ communication, WIFI communication, Radio Frequency Identification (RFID) communication, 3G, 4G, and 5G communications, and the like. However, this is merely exemplary and is not intended to be limiting.

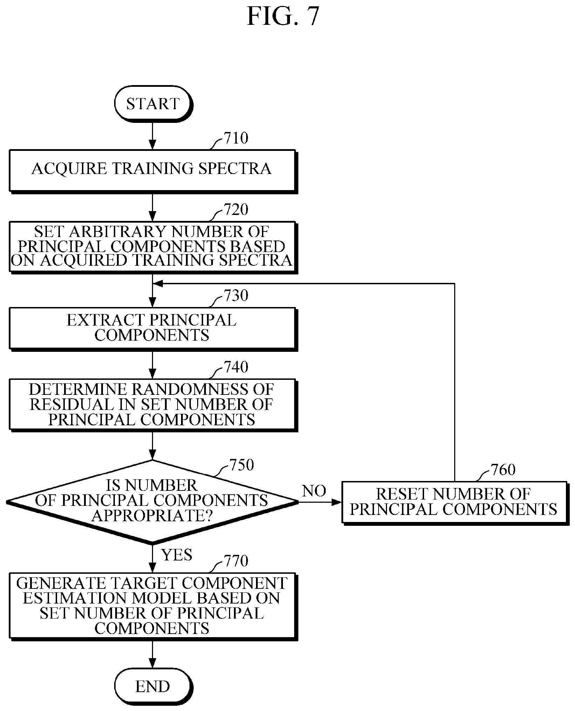

FIG. 7 is a flowchart illustrating a method of estimating a target component according to an example embodiment of the present disclosure. The method of estimating a target component in FIG. 7 may be performed by the apparatuses 100 and 600 for estimating a target component according to the embodiments of FIGS. 1 and 6.

Referring to FIG. 7, the apparatus for estimating a target component may first acquire a plurality of training spectra in operation 710. In particular, the apparatus for estimating a target component may acquire a plurality of in vivo spectra measured during a training interval, including a fasting period, in which a target component concentration of an object is substantially constant. A detailed description thereof will be omitted.

Then, the apparatus for estimating a target component may set an arbitrary number of principal components based on the acquired training spectra in operation 720.

Subsequently, the apparatus for estimating a target component may extract principal components according to the set number of principal components in operation 730. In particular, the apparatus for estimating a target component may extract the principal components by using Principal Component Analysis (PCA), Independent Component Analysis (ICA), Non-negative Matrix Factorization (NMF), Singular Value Decomposition (SVD), and the like.

Next, the apparatus for estimating a target component may determine randomness of residual in the set number of principal components in operation 740.

In particular, based on whether the residual has a predetermined pattern, the apparatus for estimating a target component may determine the randomness of the residual according to the set number of principal components. Specifically, if the residual has a predetermined pattern, the apparatus for estimating a target component may determine that the residual has no randomness; and if the residual has no predetermined pattern, the apparatus for estimating a target component may determine that the residual has randomness.

In particular, upon acquiring the respective residual spectra of the plurality of training spectra, the apparatus for estimating a target component may determine whether the residual has a pattern based on the acquired respective residual spectra. For example, the apparatus for estimating a target component may calculate a correlation matrix between wavelengths of the respective residual spectra, and may determine whether the residual has a pattern based on the calculated correlation matrix. In particular, the apparatus for estimating a target component may assign a randomness index to the calculated correlation matrix and may calculate a total index form the set number of principal components. A detailed description thereof will be omitted.

Then, the apparatus for estimating a target component may determine whether the number of principal components is appropriate in operation 750.

In particular, based on the randomness determined in operation 740, the apparatus for estimating a target component may determine whether the number of principal components is appropriate. For example, if the calculated total index for the set number of principal component is smaller than or equal to a predetermined value, the apparatus for estimating a target component may determine that the set number of principal components is appropriate; by contrast, if the calculated total index for the set number of principal component exceeds the predetermined value, the apparatus for estimating a target component may determine that the set number of principal components is inappropriate. A detailed description thereof will be omitted.

Upon determining that the number of principal components is inappropriate, the apparatus for estimating a target component may reset the number of principal components in operation 760. Then, the apparatus for estimating a target component may perform operations 730, 740, and 750 according to the reset number of principal components.

Upon determining that the number of principal components is appropriate, the apparatus for estimating a target component may generate a target component estimation model based on the set number of principal components in operation 770. In particular, the apparatus for estimating a target component may generate a target component estimation model based on linear regression, such as net analyte signal (NAS), classical least square (CLS), etc., machine learning, the Beer-Lambert law, and the like.

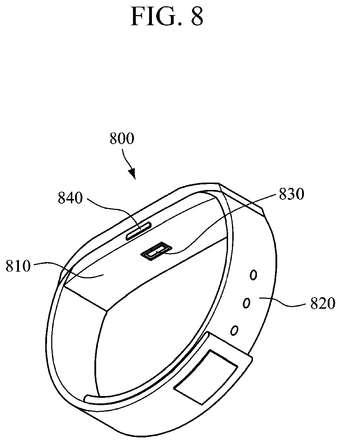

FIG. 8 is a diagram illustrating a wearable device according to an example embodiment of the present disclosure. Various examples of the aforementioned apparatuses 100 and 600 for estimating a target component may be mounted in a smartwatch wearable device as illustrated herein, but are not limited thereto and may be mounted in a portable device such as a smartphone.

Referring to FIG. 8, a wearable device 800 may include a main body 810 and a strap 820.

The strap 820 may be connected to both ends of the main body 810 so that the main body 810 may be worn on a user's wrist. The strap 820 may be flexible so as to be wrapped around the user's wrist. The strap 820 may be composed of a first strap and a second strap which are separated from each other. One ends of the first strap and the second strap are connected to both sides of the main body 810, and the other ends thereof may be connected to each other via a connecting means. In particular, the connecting means may be formed as magnetic connection, Velcro connection, pin connection, etc., but is not limited thereto. Further, the strap 820 is not limited thereto, and may be integrally formed as a non-detachable band.

The main body 810 may include various modules for various functions of the wearable device 800. A battery may be embedded in the main body 810 or the strap 820 to supply power to the various modules.

A spectrometer 830 for acquiring spectra from a user may be mounted in the main body 820. The spectrometer 830 may include a light source and a photodetector as described above, but is not limited thereto.

A processor may be mounted in the main body 810 and may be electrically connected to various components of the wearable device 800. The processor may determine whether a number of principal components is appropriate, which is set based on training spectra acquired by the spectrometer, and upon determining that the number of principal components is appropriate, the processor may generate an estimation model for estimating a target component based on NAS.

In addition, a storage may be included in the main body 810 and may store data related to a target component estimation function of the wearable device 800 and/or data related to other functions.

Further, a manipulator 840 for receiving a user's control command and transmitting the control command to the processor may be mounted on one side of the main body 810. The manipulator 840 may have a power button to input a command to turn on/off the wearable device 800.

A display for outputting information to a user may be disposed on a front surface of the main body 810. The display may include a touch screen for receiving touch input. The display may receive a user's touch input and transmit the touch input to the processor, and may display processing results of the processor.

Moreover, a communication interface for communicating with an external device may be mounted in the main body 810. The communication interface may transmit a target component estimation result to an external device, e.g., a user's smartphone, and may receive data related to estimating a target component from the external device.

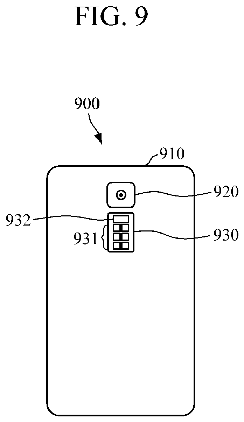

FIG. 9 is a diagram illustrating a smart device according to an example embodiment of the present disclosure. A smart device 900 may include various examples of the aforementioned apparatuses 100 and 600 for estimating a target component. The smart device may include a smartphone and a tablet PC.

Referring to FIG. 9, the smart device 900 may include a main body 910 and a spectrometer 930 mounted on one surface of the main body 910. As described above, the spectrometer 930 may acquire a plurality of training spectra from an object, and may include, for example, a light source 931 and a photodetector 932, but is not limited thereto.

A processor may be mounted in the main body 910 and may determine whether a number of principal components is appropriate, which is set based on training spectra acquired by the spectrometer 930, and upon determining that the number of principal components is appropriate, the processor may generate an estimation model for estimating a target component based on NAS by using the set number of principal components. A detailed description thereof will be omitted.

An image sensor 920 may be mounted in the main body 910 as illustrated in FIG. 9. When a user's object, e.g., a finger, approaches the spectrometer 930, the image sensor 920 may capture an image of the finger and may transmit the captured image to the processor. In particular, based on the image of the finger, the processor may identify a relative position of the finger with respect to an actual position of the spectrometer 930, and may provide a graphic object, indicating information on the relative position of the finger and the like, to a user through an output interface, or may identify a object by recognizing a fingerprint.

In addition, a storage, a communication interface, and the like may be mounted in the main body 910 to store the target component estimation model generated by the processor or to transmit the generated estimation model to an external device. Furthermore, various other modules for performing various functions may be mounted in the main body 910.

The present disclosure can be realized as a computer-readable code written on a computer-readable recording medium. The computer-readable recording medium may be any type of recording device in which data is stored in a computer-readable manner.

Examples of the computer-readable recording medium include a ROM, a RAM, a CD-ROM, a magnetic tape, a floppy disc, an optical data storage, and a carrier wave (e.g., data transmission through the Internet). The computer-readable recording medium can be distributed over a plurality of computer systems connected to a network so that a computer-readable code is written thereto and executed therefrom in a decentralized manner. Functional programs, codes, and code segments needed for realizing the present invention can be readily deduced by programmers of ordinary skill in the art to which the invention pertains.

**Claims**

1. An apparatus (100) for estimating a target component, the apparatus comprising:

   a spectrometer (110) configured to acquire training spectra; and
   a processor (120) configured to:

   extract a set number of principal components from the training spectra,
   determine whether the set number of principal components is appropriate based on randomness of residual in the set number of principal components, and

based on the set number of principal components being determined to be appropriate, generate a target component estimation model based on the set number of principal components.

2. The apparatus of claim 1, wherein the processor is further configured to extract the set number of principal components from the training spectra by using at least one of Principal Component Analysis, PCA, Independent Component Analysis, ICA, Non-negative Matrix Factorization, NMF, and Singular Value Decomposition, SVD.

3. The apparatus of claim 1 or 2, wherein the residual is a difference between the training spectra, and reconstructed spectra that are reconstructed from the training data based on the set number of principal components.

4. The apparatus of one of claims 1 to 3, wherein the processor is further configured to determine the randomness of the residual, based on a determination of whether the residual in the set number of principal components has a predetermined pattern.

5. The apparatus of claim 4, wherein:

in response to the determination that the residual in the set number of principal components has the predetermined pattern, the processor is further configured to determine that the residual has no randomness and determine that the set number of principal components is inappropriate; and
in response to the determination that the residual in the set number of principal components does not have the predetermined pattern, the processor is further configured to determine that the residual has the randomness and determines that the set number of principal components is appropriate.

6. The apparatus of claim 4 or 5, wherein the processor is further configured to acquire residual spectra by subtracting reconstructed spectra, that are reconstructed based on the set number of principal components, from the training spectra, and determine whether the residual has the predetermined pattern based on a correlation between wavelengths of the residual spectra.

7. The apparatus of claim 6, wherein the processor is further configured to calculate a correlation matrix between the wavelengths of the residual spectra and assign a randomness index to the correlation matrix.

8. The apparatus of claim 7, wherein in response to each correlation value in the correlation matrix being greater than or equal to a predetermined value, the processor is further configured to assign an index of 1, and in response to each correlation value in the correlation matrix being less than the predetermined value, the processor is further configured to assign an index of 0, or
wherein the processor is further configured to calculate a total index for the set number of principal components based on the randomness index, and in response to the total index being less than or equal to a predetermined value, the processor is further configured to determine that the set number of principal components is appropriate.

9. The apparatus of one of claims 1 to 8, wherein based on a determination that the set number of principal components is inappropriate, the processor is further configured to change the set number of principal components.

10. The apparatus of one of claims 1 to 9, wherein by using a determined optimal number of principal components, the processor is further configured to generate the target component estimation model based on Net Analyte Signal, NAS, calculation.

11. The apparatus of one of claims 1 to 10, wherein the spectrometer is further configured to acquire the training spectra during a training interval, comprising a fasting period of a user.

12. The apparatus of one of claims 1 to 11, wherein the spectrometer is further configured to acquire an estimation spectrum, and the processor is further configured to estimate the target component by using the target component estimation model and the estimation spectrum, and
wherein the target component comprises at least one of glucose, urea, lactate, triglyceride, total protein, cholesterol, and ethanol.

13. A computer-readable recording medium storing instructions that, when executed by a processor of an apparatus for estimating a target biological component having a spectrometer configured to acquire spectra from an object, cause the processor to execute the instructions to:

input the spectra to a target component estimation model, wherein the target component estimation model is trained using a number of principal components that are extracted from training spectra and satisfies a preset randomness criterion of residual; and

obtain, as output of the target biological component, an estimated value of the target biological component.

14. A method of estimating a target component, the method comprising:

acquiring (710) training spectra;

extracting (730) a set number of principal components from the training spectra;

determining (740) whether the set number of principal components is appropriate based on randomness of residual in the set number of principal components; and

based on the set number of principal components being determined to be appropriate, generating (770) a target component estimation model based on the set number of principal components.

15. The method of claim 14 adapted to operate the apparatus for estimating a target component according to one of claims 1 to 12.

**Patentansprüche**

1. Vorrichtung (100) zum Schätzen einer Zielkomponente, wobei die Vorrichtung umfasst:

ein Spektrometer (110), ausgebildet zum Erfassen von Trainingsspektren; und

einen Prozessor (120), ausgebildet zum:

Extrahieren einer festgelegten Zahl von Hauptkomponenten aus den Trainingsspektren,

Bestimmen, ob die Zahl von Hauptkomponenten passend ist, basierend auf der Zufälligkeit des Rests in der festgelegten Zahl von Hauptkomponenten, und

auf der Basis der festgelegten Zahl von Hauptkomponenten, die als passend bestimmt wurde, Erzeugen eines Zielkomponentenschätzmodells auf der Basis der festgelegten Zahl von Hauptkomponenten.

2. Vorrichtung nach Anspruch 1, wobei der Prozessor ferner ausgebildet ist zum Extrahieren der festgelegten Zahl von Hauptkomponenten aus den Trainingsspektren durch Verwenden von einem Element der Gruppe umfassend Principal Component Analysis (PCA), Independent Component Analysis (ICA), Non-negative Matrix Factorization (NMF) und Singular Value Decomposition (SVD).

3. Vorrichtung nach Anspruch 1 oder 2, wobei der Rest eine Differenz zwischen den Trainingsspektren und rekonstruierten Spektren ist, die aus den Trainingsdaten auf der Basis der festgelegten Zahl von Hauptkomponenten rekonstruiert sind.

4. Vorrichtung nach einem der Ansprüche 1 bis 3, wobei der Prozessor ferner zum Bestimmen der Zufälligkeit des Rests auf der Basis einer Bestimmung, ob der Rest in der festgelegten Zahl von Hauptkomponenten ein vorbestimmtes Muster aufweist, ausgebildet ist.

5. Vorrichtung nach Anspruch 4, wobei:

als Reaktion auf das Bestimmen, dass der Rest in der festgelegten Zahl von Hauptkomponenten das vorbestimmte Muster aufweist, der Prozessor ferner zum Bestimmen, dass der Rest keine Zufälligkeit aufweist, und Bestimmen, dass die festgelegte Zahl von Hauptkomponenten unpassend ist, ausgebildet ist; und

als Reaktion auf das Bestimmen, dass der Rest in der festgelegten Zahl von Hauptkomponenten das vorbestimmte Muster nicht aufweist, der Prozessor ferner zum Bestimmen, dass der Rest die Zufälligkeit aufweist, und Bestimmen, dass die festgelegte Zahl von Hauptkomponenten passend ist, ausgebildet ist.

6. Vorrichtung nach Anspruch 4 oder 5, wobei der Prozessor ferner zum Erfassen von Restspektren durch Subtrahieren von rekonstruierten Spektren, die auf der Basis der festgelegten Zahl von Hauptkomponenten rekonstruiert sind, von den Trainingsspektren und Bestimmen, ob der Rest das vorbestimmte Muster aufweist, auf der Basis einer Korrelation zwischen Wellenlängen der Restspektren ausgebildet ist.

7. Vorrichtung nach Anspruch 6, wobei der Prozessor ferner zum Berechnen einer Korrelationsmatrix zwischen den Wellenlängen der Restspektren und Zuweisen eines Zufallsindex zur Korrelationsmatrix ausgebildet ist.

8. Vorrichtung nach Anspruch 7, wobei als Reaktion darauf, dass jeder Korrelationswert in der Korrelationsmatrix größer gleich einem vorbestimmten Wert ist, der Prozessor ferner zum Zuweisen eines Index von 1 und als Reaktion darauf, dass jeder Korrelationswert in der Korrelationsmatrix kleiner als der vorbestimmte Wert ist, der Prozessor ferner zum Zuweisen eines Index von 0 ausgebildet ist, oder
wobei der Prozessor ferner zum Berechnen eines Gesamtindex für die festgelegte Zahl von Hauptkomponenten auf der Basis des Zufälligkeitsindex ausgebildet ist und als Reaktion darauf, dass der Gesamtindex kleiner gleich einem vorbestimmten Wert ist, der Prozessor ferner zum Bestimmen, dass die festgelegte Zahl von Hauptkomponenten passend ist, ausgebildet ist.

9. Vorrichtung nach einem der Ansprüche 1 bis 8, wobei auf der Basis eines Bestimmens, dass die festgelegte Zahl von Hauptkomponenten unpassend ist, der Prozessor ferner zum Ändern der festgelegten Zahl von Hauptkomponenten ausgebildet ist.

10. Vorrichtung nach einem der Ansprüche 1 bis 9, wobei durch Verwenden einer bestimmten optimalen Zahl von Hauptkomponenten der Prozessor ferner zum Erzeugen des Zielkomponentenschätzmodells auf der Basis einer Net-Analyte-Signal-(NAS-)Berechnung ausgebildet ist.

11. Vorrichtung nach einem der Ansprüche 1 bis 10, wobei das Spektrometer ferner zum Erfassen der Trainingsspektren während eines Trainingsintervalls, umfassend eine Fastenperiode eines Benutzers, ausgebildet ist.

12. Vorrichtung nach einem der Ansprüche 1 bis 11, wobei das Spektrometer ferner zum Erfassen eines Schätzspektrum ausgebildet ist und der Prozessor ferner zum Schätzen der Zielkomponente durch Verwenden des Zielkomponentenschätzmodells und des Schätzspektrums ausgebildet ist, und
wobei die Zielkomponente wenigstens ein Element der Gruppe umfassend Glukose, Harnstoff, Laktat, Triglycerid, Gesamtprotein, Cholesterin und Ethanol umfasst.

13. Computerlesbares Aufzeichnungsmedium zum Speichern von Anweisungen, die, wenn von einem Prozessor einer Vorrichtung zum Schätzen einer biologischen Zielkomponente mit einem Spektrometer, ausgebildet zum Erfassen von Spektren von einem Objekt, ausgeführt, den Prozessor veranlassen, die Anweisungen auszuführen zum:

    Eingeben der Spektren in ein Zielkomponentenschätzmodell, wobei das Zielkomponentenschätzmodell unter Verwendung einer Zahl von Hauptkomponenten trainiert wird, die aus Trainingsspektren extrahiert werden und ein vorgegebenes Zufälligkeitskriterium des Rests erfüllen; und
    Erhalten eines geschätzten Werts der biologischen Zielkomponente als Ausgabe der biologischen Zielkomponente.

14. Verfahren zum Schätzen einer Zielkomponente, wobei das Verfahren umfasst: Erfassen (710) von Trainingsspektren;

    Extrahieren (730) einer festgelegten Zahl von Hauptkomponenten aus den Trainingsspektren,
    Bestimmen (740), ob die Zahl von Hauptkomponenten passend ist, basierend auf der Zufälligkeit des Rests in der festgelegten Zahl von Hauptkomponenten; und
    auf der Basis der festgelegten Zahl von Hauptkomponenten, die als passend bestimmt wurde, Erzeugen (770) eines Zielkomponentenschätzmodells auf der Basis der festgelegten Zahl von Hauptkomponenten.

15. Verfahren nach Anspruch 14, angepasst zum Betreiben der Vorrichtung zum Schätzen einer Zielkomponente nach einem der Ansprüche 1 bis 12.


**Revendications**

1. Appareil (100) pour estimer une composante cible, l'appareil comprenant :

    un spectromètre (110) configuré pour acquérir des spectres d'entraînement ; et
    un processeur (120) configuré pour :

extraire un nombre établi de composantes principales à partir des spectres d'entraînement,

déterminer si le nombre établi de composantes principales est approprié sur la base d'un caractère aléatoire de résidu dans le nombre établi de composantes principales, et

sur la base de la détermination que le nombre établi de composantes principales est approprié, générer un modèle d'estimation de composante cible sur la base du nombre établi de composantes principales.

2. Appareil selon la revendication 1, dans lequel le processeur est en outre configuré pour extraire le nombre établi de composantes principales à partir des spectres d'entraînement en utilisant au moins l'une parmi une analyse en composantes principales, PCA, une analyse en composantes indépendantes, ICA, une factorisation par matrices non-négatives, NMF, et une décomposition en valeurs singulières, SVD.

3. Appareil selon la revendication 1 ou 2, dans lequel le résidu est une différence entre les spectres d'entraînement et des spectres reconstruits qui sont reconstruits à partir des données d'entraînement sur la base du nombre établi de composantes principales.

4. Appareil selon l'une des revendications 1 à 3, dans lequel le processeur est en outre configuré pour déterminer le caractère aléatoire du résidu, sur la base d'une détermination déterminant si le résidu dans le nombre défini de composantes principales a un motif prédéterminé.

5. Appareil selon la revendication 4, dans lequel :

en réponse à la détermination que le résidu dans le nombre établi de composantes principales a le motif prédéterminé, le processeur est en outre configuré pour déterminer que le résidu n'a aucun caractère aléatoire et déterminer que le nombre établi de composantes principales est inapproprié ; et

en réponse à la détermination que le résidu dans le nombre établi de composantes principales n'a pas le motif prédéterminé, le processeur est en outre configuré pour déterminer que le résidu a le caractère aléatoire et détermine que le nombre établi de composantes principales est approprié.

6. Appareil selon la revendication 4 ou 5, dans lequel le processeur est en outre configuré pour acquérir des spectres résiduels en soustrayant des spectres reconstruits, qui sont reconstruits sur la base du nombre établi de composantes principales, à partir des spectres d'entraînement, et déterminer si le résidu a le motif prédéterminé sur la base d'une corrélation entre des longueurs d'onde des spectres résiduels.

7. Appareil selon la revendication 6, dans lequel le processeur est en outre configuré pour calculer une matrice de corrélation entre les longueurs d'onde des spectres résiduels et attribuer un indice de caractère aléatoire à la matrice de corrélation.

8. Appareil selon la revendication 7, dans lequel, en réponse au fait que chaque valeur de corrélation dans la matrice de corrélation est supérieure ou égale à une valeur prédéterminée, le processeur est en outre configuré pour attribuer un indice de 1, et en réponse au fait que chaque valeur de corrélation dans la matrice de corrélation est inférieure à la valeur prédéterminée, le processeur est en outre configuré pour attribuer un indice de 0, ou

dans lequel le processeur est en outre configuré pour calculer un indice total pour le nombre établi de composantes principales sur la base de l'indice de caractère aléatoire, et en réponse au fait que l'indice total est inférieur ou égal à une valeur prédéterminée, le processeur est en outre configuré pour déterminer que le nombre établi de composantes principales est approprié.

9. Appareil selon l'une des revendications 1 à 8, dans lequel, sur la base d'une détermination déterminant que le nombre établi de composantes principales est inapproprié, le processeur est en outre configuré pour changer le nombre établi de composantes principales.

10. Appareil selon l'une des revendications 1 à 9, dans lequel, en utilisant un nombre optimal déterminé de composantes principales, le processeur est en outre configuré pour générer le modèle d'estimation de composante cible sur la base d'un calcul de signal analyte net, NAS.

11. Appareil selon l'une des revendications 1 à 10, dans lequel le spectromètre est en outre configuré pour acquérir les spectres d'entraînement pendant un intervalle d'entraînement, comprenant une période de jeûne d'un utilisateur.

12. Appareil selon l'une des revendications 1 à 11, dans lequel le spectromètre est en outre configuré pour acquérir un spectre d'estimation, et le processeur est en outre configuré pour estimer la composante cible en utilisant le modèle d'estimation de composante cible et le spectre d'estimation, et

dans lequel la composante cible comprend au moins l'un parmi glucose, urée, lactate, triglycéride, protéine totale, cholestérol et éthanol.

13. Support d'enregistrement lisible par ordinateur stockant des instructions qui, lorsqu'elles sont exécutées par un processeur d'un appareil d'estimation d'une composante biologique cible comprenant un spectromètre configuré pour acquérir des spectres d'un objet, amènent le processeur à exécuter les instructions pour :

entrer les spectres dans un modèle d'estimation de composante cible, dans lequel le modèle d'estimation de composante cible est entraîné en utilisant un nombre de composantes principales qui sont extraites de spectres d'entraînement et satisfait à un critère de caractère aléatoire préétabli de résidu ; et

obtenir, en tant que sortie de la composante biologique cible, une valeur estimée de la composante biologique cible.

14. Procédé d'estimation d'une composante cible, le procédé comprenant :

l'acquisition (710) de spectres d'entraînement ;

l'extraction (730) d'un nombre établi de composantes principales à partir des spectres d'entraînement ;

la détermination (740) déterminant si le nombre établi de composantes principales est approprié sur la base d'un caractère aléatoire de résidu dans le nombre établi de composantes principales ; et

sur la base de la détermination que le nombre établi de composantes principales est approprié, la génération (770) d'un modèle d'estimation de composante cible sur la base du nombre établi de composantes principales.

15. Procédé selon la revendication 14 adapté pour faire fonctionner l'appareil d'estimation d'une composante cible selon l'une des revendications 1 à 12.

FIG. 1

# FIG. 2

Glucose (mM)

# FIG. 3A

(1)

(2)

# FIG. 3B

(1)

(2)

# FIG. 4A

(1)

(2)

# FIG. 4B

(1)

(2)

# FIG. 5A

WAVELENGTH (nm)

WAVELENGTH (nm)

FIG. 5B

# FIG. 6

# FIG. 7

```
        ┌─────────┐
        │  START  │
        └────┬────┘
             │
             ▼                    710
┌──────────────────────────────┐
│   ACQUIRE TRAINING SPECTRA    │
└──────────────┬───────────────┘
               │                  720
┌──────────────────────────────┐
│   SET ARBITRARY NUMBER OF     │
│  PRINCIPAL COMPONENTS BASED   │
│  ON ACQUIRED TRAINING SPECTRA │
└──────────────┬───────────────┘
               │                  730
┌──────────────────────────────┐
│     EXTRACT PRINCIPAL         │
│       COMPONENTS              │
└──────────────┬───────────────┘
               │                  740
┌──────────────────────────────┐
│  DETERMINE RANDOMNESS OF      │
│  RESIDUAL IN SET NUMBER OF    │
│   PRINCIPAL COMPONENTS        │
└──────────────┬───────────────┘
               │                  750
         ◇─────────────◇   NO    ┌────────────────────────┐
         │ IS NUMBER    │───────▶│   RESET NUMBER OF       │
         │OF PRINCIPAL  │        │ PRINCIPAL COMPONENTS    │  760
         │ COMPONENTS   │        └────────────────────────┘
         │ APPROPRIATE? │
         ◇──────┬──────◇
                │ YES             770
┌──────────────────────────────┐
│ GENERATE TARGET COMPONENT     │
│ ESTIMATION MODEL BASED ON     │
│  SET NUMBER OF PRINCIPAL      │
│       COMPONENTS              │
└──────────────┬───────────────┘
               │
          ┌────▼────┐
          │   END   │
          └─────────┘
```

# FIG. 8

FIG. 9

## REFERENCES CITED IN THE DESCRIPTION

**Non-patent literature cited in the description**

- Principal component analysis-adaptive neuro-fuzzy inference systems (ANFISs) for the simultaneous spectrophotometric determination of three metals in water samples. **GOODARZI, M. et al.** SPECTRO-CHIMICA ACTA PART A: MOLECULAR AND BIO-MOLECULAR SPECTROSCOPY. Elsevier, 15 August 2009, vol. 73, 608-614

- **RUTLEDGE, D. N. et al.** Durbin-Watson statistic as a morphological estimator of information content. *ANALYTICA CHIMICA ACTA*, 01 January 2002, vol. 454, 277-295